# EUROPEAN PATENT APPLICATION

(11) **EP 4 692 054 A1**
(43) Date of publication of application: **11.02.2026**
(21) Application number: 24780171.5
(22) Date of filing: 25.03.2024
(51) Int. Cl.: C07C 321/04, C08G 59/66

(54) **THIOL COMPOUND**

(30) Priority: 31.03.2023 JP 2023057964
(71) Applicant: AJINOMOTO CO., INC., Chuo-ku Tokyo 104-8315 (JP)
(72) Inventor: OGINO, Keiji, Kawasaki-shi, Kanagawa 210-0801 (JP); SATO, Naoya, Kawasaki-shi, Kanagawa 210-0801 (JP); KUBO, Yuki, Kawasaki-shi, Kanagawa 210-0801 (JP); TOMINAGA, Taiga, Kawasaki-shi, Kanagawa 210-0801 (JP)
(74) Representative: Mewburn Ellis LLP
(86) International application number: PCT/JP2024/011677
(87) International publication number: WO 2024/204053

(57) **Abstract**

The present invention is to provide a novel thiol compound used for curing of epoxy resins, etc. Specifically, the present invention is to provide a compound represented by the general formula (I), a curing agent for epoxy resins, and an epoxy resin composition comprising the compound of formula (I).

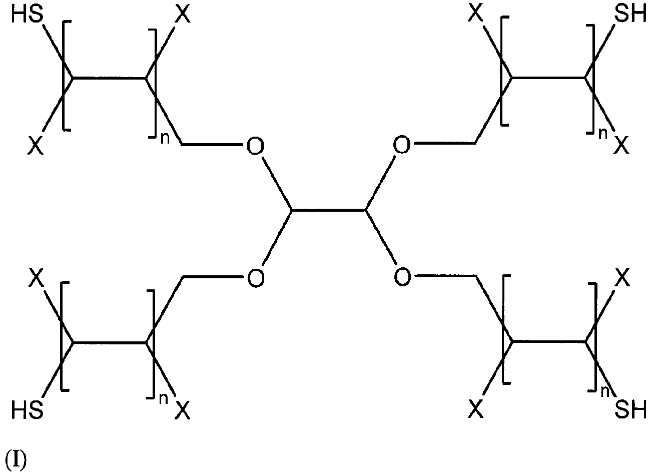

## Description

### Technical Field

The present invention relates to a novel thiol compound used for curing of epoxy resins, etc. More specifically, the said novel thiol compound can be used as a curing agent for epoxy resins.

### Background Art

Compositions using a compound containing a thiol group (also called sulfanyl group or mercapto group, -SH group) as a curing agent for epoxy resins are excellent in low-temperature curability, therefore various investigations have been made. In particular, compounds having a plurality of thiol groups within the molecule have been subjected to various research (Patent Literatures 1 to 3).

### Citation List

### Patent Literatures

Patent Literature 1: Japanese Unexamined Patent Application Publication No. 2012-153794
Patent Literature 2: Japanese Unexamined Patent Application Publication No. 2016-164134
Patent Literature 3: International Publication No. WO 2014/084292 Pamphlet

### Summary of Invention

Generally, at the time of curing epoxy resins, by using a bifunctional thiol compound with a long molecular chain, it is possible to obtain a cured product having high elongation and low elasticity, excellent in impact resistance. However, when a finger or the like touched the surface of a cured product for which curing had progressed, an uncured cured product adhered to the finger or the like, and there were cases where the surface state of the cured product was affected. Accordingly, a thiol compound that does not cause such adhesion to fingers or the like, exhibits so-called tack-free performance, and can develop high elongation and low elasticity has been demanded.

The present inventors conducted diligent investigations in order to solve the above-mentioned problem. Then, the present inventors found that a compound having a specific thiol group, while being a polyfunctional thiol compound that develops high elongation and low elasticity, is effective as a curing agent that provides a resin cured product having good tack-free performance, leading to the present invention.

That is, the present invention can include the following aspects.
[1] A compound represented by the general formula (I): 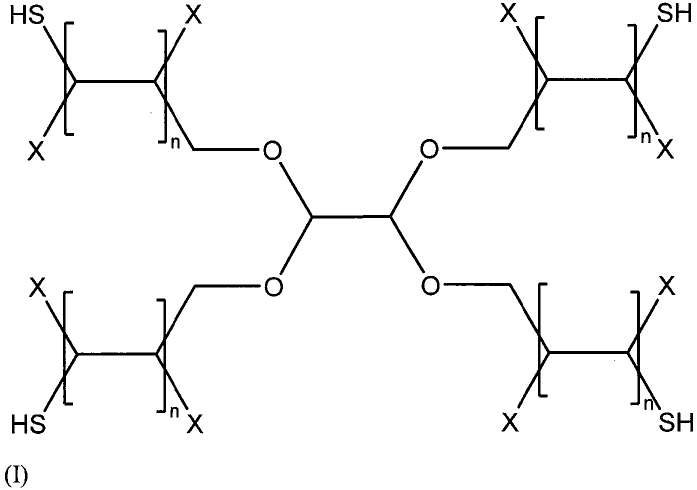 (in the formula (I), X is, each independently, a hydrogen atom, a C1-C6 alkyl group, a C1-C6 haloalkyl group, or a C1-C6 alkoxy group, and n is, each independently, an integer of 1 to 5).
[2] The compound according to [1], wherein X is, each independently, a hydrogen atom, a methyl group, or an ethyl group, and n is an integer of 1 to 3.
[3] The compound according to [1], wherein X is, each independently, a hydrogen atom or a methyl group, and n is 1.
[4] A compound shown below. 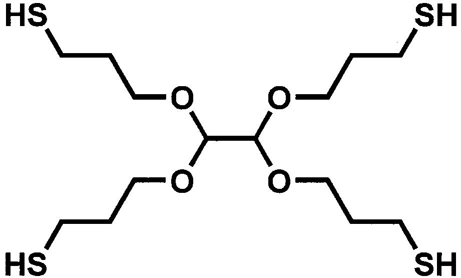 or 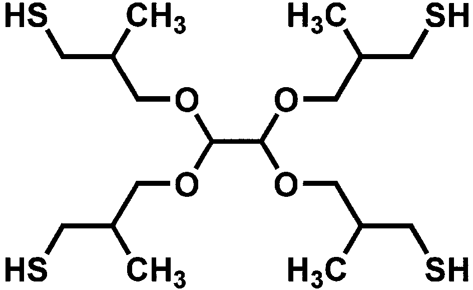
[5] An epoxy resin composition, comprising an epoxy resin and the compound according to any one of [1] to [4].
[6] A curing agent for epoxy resins, comprising the compound according to any one of [1] to [4].
[7] An epoxy resin cured product, formed by thermosetting an epoxy resin and the compound according to any one of [1] to [4].

According to the present invention, it is possible to provide a curing agent for epoxy resins which, while being a curing agent for epoxy resins capable of developing high elongation and low elasticity, provides a cured product having good tack-free performance. By using the compound having a thiol group of the present invention, curing of the epoxy resin proceeds rapidly, and it is possible to provide an epoxy resin cured product having good tack-free properties.

### Brief Description of Drawings

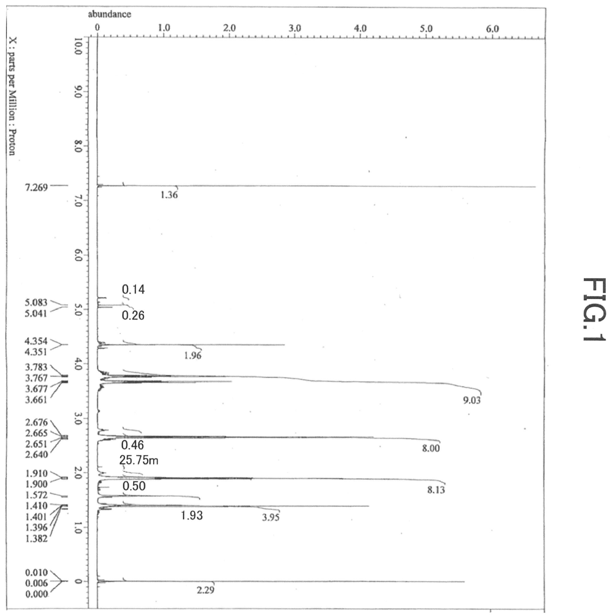

Fig. 1 shows NMR data of the thiol compound (1) prepared in Example 1.

### Description of Embodiments

### [Thiol Compound]

One aspect of the present invention is a thiol compound represented by the following general formula (I).

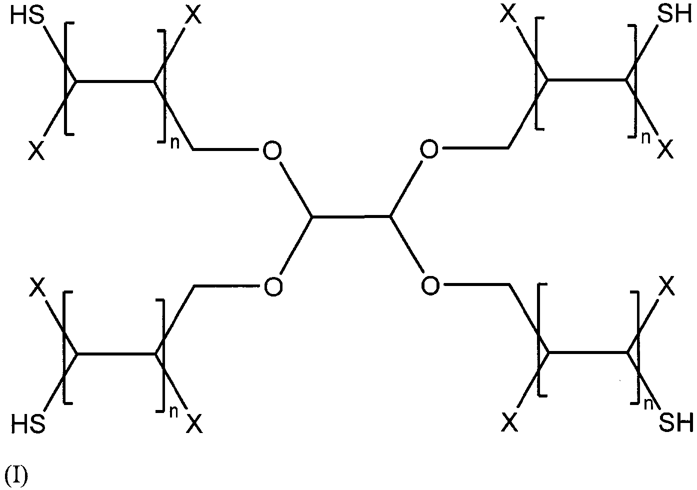

In formula (I), X is, each independently, a hydrogen atom, a C1-C6 alkyl group, a C1-C6 haloalkyl group, or a C1-C6 alkoxy group, preferably a hydrogen atom or a C1-C6 alkyl group, more preferably a hydrogen atom, a methyl group, or an ethyl group, still more preferably a hydrogen atom or a methyl group.

n is, each independently, an integer of 1 to 5, preferably n is an integer of 1 to 3, more preferably n is 1.

Another aspect of the present invention is a thiol compound represented by the following general formula (Ib).

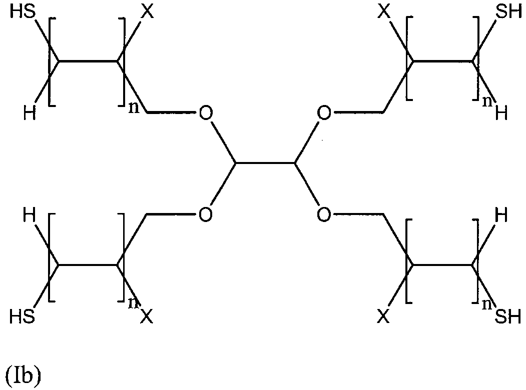

In formula (Ib), the definitions of X and n are the same as the definitions in formula (I).

As more specific aspects of the thiol compound of the present invention, the following thiol compounds (1) and (2) can be mentioned.

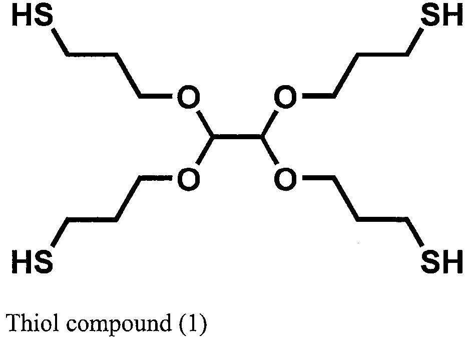

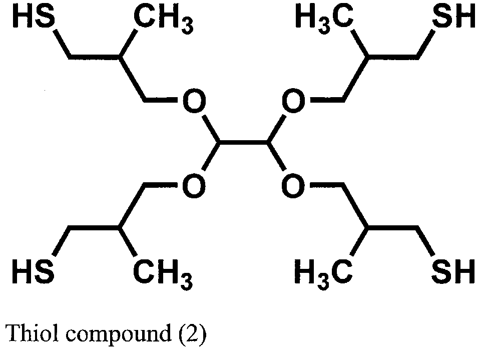

### [Method for Preparing Thiol Compound]

The above-mentioned thiol compound can be produced by a known method, but can be prepared, for example, as follows. Hereinafter, explanation will be given taking the above-mentioned thiol compound (1) as an example.

First, using tetraallyloxyethane as a starting material, an organic solvent such as toluene and azobisisobutyronitrile are added, further thioacetic acid is added, and reacted, for example, at 50 to 200°C, preferably 60 to 150°C, more preferably 80°C±10 to 20°C, for example, for 30 minutes to 12 hours, preferably 1 to 5 hours, more preferably 3 hours±1 hour. An organic solvent such as toluene is removed from the obtained reaction product to obtain tetraallyloxyethane (thioester form). The obtained thioester form is reacted together with an alcohol such as methanol and a base such as sodium hydroxide, for example, at 50 to 200°C, preferably 60 to 150°C, more preferably 70°C±10 to 20°C, for example, for 30 minutes to 12 hours, preferably 1 to 5 hours, more preferably 1.5 hours±1 to 2 hours. The obtained reaction product is neutralized with hydrochloric acid or the like, and extraction is performed 1 to 5 times, preferably about 3 times, using an organic solvent such as toluene. An organic solvent such as toluene is removed from the obtained organic solvent layer, and thiol compound (1) is obtained from the said organic solvent layer.

### [Uses of Thiol Compound]

The above-mentioned thiol compound is a polyfunctional thiol compound having a long molecular chain and having four or more thiol groups, and by using it as a curing agent during the curing of an epoxy resin, it is possible to obtain a cured product having high elongation and low elasticity, excellent in impact resistance. Further, the said thiol compound is useful as a curing agent for epoxy resins because it has characteristics of being excellent in low-temperature curability and, compared to other thiol compounds having an ester group, being excellent in hydrolysis resistance. Moreover, the above-mentioned thiol compound can also be used as a crosslinking agent or a curing aid for compounds having a carbon-carbon double bond (ene compounds). The reaction between a thiol compound and a carbon-carbon double bond is known as a thiol-ene reaction, and the above-mentioned thiol compound can be used in the reaction (thiol-ene reaction) between a resin having a carbon-carbon double bond, such as a (meth)acrylate compound, an allyl compound, a vinyl compound, an unsaturated polyester, or polybutadiene, and the above-mentioned thiol compound. By using the said thiol compound as a crosslinking agent or a curing aid, the said thiol compound is less susceptible to oxygen inhibition during reaction and is excellent in reactivity with compounds having a carbon-carbon double bond, therefore crosslinking of the compound having a carbon-carbon double bond can be rapidly advanced. In addition, in each application, the thiol compound of the present invention may be used alone as one type, or may be used in combination of multiple types.

### [Epoxy Resin Composition]

One aspect of the present invention is an epoxy resin composition, comprising an epoxy resin and the above-mentioned thiol compound.

As the epoxy resin, for example, an epoxy resin compound having two or more epoxy groups per molecule on average can be mentioned. Examples of the epoxy resin include polyglycidyl ethers obtained by reacting polyhydric phenols such as bisphenol A, bisphenol F, bisphenol AD, catechol, and resorcinol, or polyhydric alcohols such as glycerin and polyethylene glycol, with epichlorohydrin; polyglycidyl ether esters obtained by reacting hydroxy acids such as p-hydroxybenzoic acid and β-hydroxynaphthoic acid with epichlorohydrin; polyglycidyl esters obtained by reacting polycarboxylic acids such as phthalic acid and terephthalic acid with epichlorohydrin; furthermore, epoxidized phenol novolac resins, epoxidized cresol novolac resins, epoxidized polyolefins, cycloaliphatic epoxy resins, other urethane-modified epoxy resins; and the like can be mentioned.

As suitable epoxy resins, from the viewpoint of maintaining high heat resistance and low moisture permeability, etc., bisphenol A type epoxy resins, bisphenol F type epoxy resins, phenol novolac type epoxy resins, biphenyl aralkyl type epoxy resins, phenol aralkyl type epoxy resins, aromatic glycidyl amine type epoxy resins, and epoxy resins having a dicyclopentadiene structure are preferable, bisphenol A type epoxy resins and bisphenol F type epoxy resins are more preferable, and bisphenol A type epoxy resins are still more preferable.

The epoxy resin may be liquid or solid. Further, as the epoxy resin used here, a mixture of a liquid epoxy resin and a solid epoxy resin may be used. Here, "liquid" and "solid" refer to the state of the epoxy resin at room temperature (25°C).

Specific examples of liquid epoxy resins include, for example, liquid bisphenol A type epoxy resin ("jER (registered trademark) Epoxy Resin 828," "jER (registered trademark) Epoxy Resin 827" manufactured by Mitsubishi Chemical Corporation), liquid bisphenol F type epoxy resin ("jER (registered trademark) Epoxy Resin 807" manufactured by Mitsubishi Chemical Corporation), naphthalene type difunctional epoxy resin ("HP4032," "HP4032D" manufactured by DIC Corporation), liquid bisphenol AF type epoxy resin ("ZX1059" manufactured by Nippon Steel Epoxy Manufacturing Co., Ltd.), and epoxy resin with a hydrogenated structure ("jER (registered trademark) Epoxy Resin YX8000" manufactured by Mitsubishi Chemical Corporation). Among these, "jER (registered trademark) Epoxy Resin 828," "jER (registered trademark) Epoxy Resin 827," and "jER (registered trademark) Epoxy Resin 807" manufactured by Mitsubishi Chemical Corporation, which have high heat resistance and low viscosity, are preferable, and "jER (registered trademark) Epoxy Resin 828" is more preferable. Further, specific examples of solid epoxy resins include naphthalene type tetrafunctional epoxy resin ("HP4700" manufactured by DIC Corporation), dicyclopentadiene type polyfunctional epoxy resin ("HP7200" manufactured by DIC Corporation), naphthol type epoxy resin ("ESN-475V" manufactured by Nippon Steel Epoxy Manufacturing Co., Ltd. (Tohto Kasei Co., Ltd.)), epoxy resin having a butadiene structure ("PB-3600" manufactured by Daicel Corporation), epoxy resin having a biphenyl structure ("NC3000H," "NC3000L" manufactured by Nippon Kayaku Co., Ltd., "YX4000" manufactured by Mitsubishi Chemical Corporation), and the like.

From the viewpoint of coating properties, processability, and adhesiveness, it is preferable that at least 10% by mass or more of the entire epoxy resin used is a liquid epoxy resin, more preferably 30% by mass or more, and still more preferably 50% by mass or more.

In the epoxy resin composition of the present invention, the content of the thiol compound of the present invention relative to 100 parts by mass of the epoxy resin depends also on the number of thiol groups, but, for example, when the thiol compound of the present invention is a tetrafunctional thiol compound having four thiol groups (thiol equivalent=4), it is preferably 1 part by mass or more, more preferably 5 parts by mass or more, still more preferably 10 parts by mass or more, even more preferably 15 parts by mass or more, particularly preferably 20 parts by mass or more, and especially preferably 25 parts by mass or more. Further, the content of the thiol compound of the present invention relative to 100 parts by mass of the epoxy resin is preferably 95 parts by mass or less, more preferably 90 parts by mass or less, still more preferably 85 parts by mass or less, even more preferably 80 parts by mass or less, particularly preferably 75 parts by mass or less, and especially preferably 70 parts by mass or less. The preferred content of the thiol compound of the present invention may be within a range selected from any value among the said upper limit values, lower limit values, and values in the Examples. When the number of thiol groups contained in the thiol compound of the present invention becomes greater than 4, the content may be the above-mentioned content or a content proportional to the thiol equivalent. That is, for example, when the number of thiol groups is 8 (thiol equivalent=8), the above "preferably 5 parts by mass or more" may be read as "preferably 2.5 parts by mass or more," and "preferably 300 parts by mass or less" may be read as "preferably 150 parts by mass or less."

As other components that can be added to the epoxy resin composition of the present invention, curing accelerators, solvents, storage stabilizers, other curing agents, thixotropy-imparting agents, fillers, diluents, dispersants, flexibility-imparting agents, coupling agents, antioxidants, and the like can be mentioned.

As the curing accelerator, for example, a latent curing accelerator can be mentioned. A latent curing accelerator means a compound that is a solid compound insoluble in epoxy resin at room temperature (25°C), but solubilizes upon heating and functions as a curing accelerator for the epoxy resin. Imidazole compounds that are solid at room temperature (25°C) and amine adduct-based latent curing accelerators can be mentioned, but it is not limited thereto. Among these, amine adduct-based latent curing accelerators are preferable. Examples of amine adduct-based latent curing accelerators include reaction products of amine compounds and epoxy compounds (amine-epoxy adduct-based latent curing accelerators), reaction products of amine compounds and isocyanate compounds (amine-isocyanate-based latent curing accelerators), and the like.

As the imidazole compound that is solid at room temperature (25°C), for example, 2-heptadecylimidazole, 2-phenyl-4,5-dihydroxymethylimidazole, 2-undecylimidazole, 2-phenyl-4-methyl-5-hydroxymethylimidazole, 2-phenyl-4-benzyl-5-hydroxymethylimidazole, 2,4-diamino-6-(2-methylimidazolyl-(1))-ethyl-S-triazine, 2,4-diamino-6-(2'-methylimidazolyl-(1)')-ethyl-S-triazine/isocyanuric acid adduct, 2-methylimidazole, 2-phenylimidazole, 2-phenyl-4-methylimidazole, 1-cyanoethyl-2-phenylimidazole, 1-cyanoethyl-2-methylimidazole-trimellitate, 1-cyanoethyl-2-phenylimidazole-trimellitate, N-(2-methylimidazolyl-1-ethyl)-urea, and the like can be mentioned.

As the epoxy compound used as one of the production raw materials for the amine-epoxy adduct-based latent curing accelerator, for example, polyglycidyl ethers obtained by reacting polyhydric phenols such as bisphenol A, bisphenol F, catechol, and resorcinol, or polyhydric alcohols such as glycerin and polyethylene glycol, with epichlorohydrin; glycidyl ether esters obtained by reacting hydroxy acids such as p-hydroxybenzoic acid and β-hydroxynaphthoic acid with epichlorohydrin; polyglycidyl esters obtained by reacting polycarboxylic acids such as phthalic acid and terephthalic acid with epichlorohydrin; glycidylamine compounds obtained by reacting 4,4'-diaminodiphenylmethane or m-aminophenol, etc., with epichlorohydrin; furthermore, polyfunctional epoxy compounds such as epoxidized phenol novolac resins, epoxidized cresol novolac resins, and epoxidized polyolefins, or monofunctional epoxy compounds such as butyl glycidyl ether, phenyl glycidyl ether, and glycidyl methacrylate; and the like can be mentioned.

The amine compound used as a production raw material for the amine adduct-based latent curing accelerator may be any compound having one or more active hydrogens in the molecule capable of undergoing addition reaction with an epoxy group or an isocyanate group (alias: isocyanato group), and having one or more amino groups (at least one of primary amino group, secondary amino group, and tertiary amino group) in the molecule. As such amine compounds, for example, aliphatic amine compounds such as diethylenetriamine, triethylenetetramine, propylamine, 2-hydroxyethylaminopropylamine, cyclohexylamine, and 4,4'-diamino-dicyclohexylmethane; aromatic amine compounds such as 4,4'-diaminodiphenylmethane and 2-methylaniline; nitrogen atom-containing heterocyclic compounds such as 2-ethyl-4-methylimidazole, 2-ethyl-4-methylimidazoline, 2,4-dimethylimidazoline, piperidine, and piperazine; and the like can be mentioned.

Further, by particularly using a compound having a tertiary amino group in the molecule among the above-mentioned raw materials, a latent curing accelerator having excellent curing acceleration ability can be produced. As compounds having a tertiary amino group in the molecule, for example, amines having a tertiary amino group in the molecule such as dimethylaminopropylamine, diethylaminopropylamine, dipropylaminopropylamine, dibutylaminopropylamine, dimethylaminoethylamine, diethylaminoethylamine, N-methylpiperazine, 2-methylimidazole, 2-ethylimidazole, 2-ethyl-4-methylimidazole, and 2-phenylimidazole; alcohols, phenols, thiols, carboxylic acids, and hydrazides having a tertiary amino group in the molecule such as 2-dimethylaminoethanol, 1-methyl-2-dimethylaminoethanol, 1-phenoxymethyl-2-dimethylaminoethanol, 2-diethylaminoethanol, 1-butoxymethyl-2-dimethylaminoethanol, 1-(2-hydroxy-3-phenoxypropyl)-2-methylimidazole, 1-(2-hydroxy-3-phenoxypropyl)-2-ethyl-4-methylimidazole, 1-(2-hydroxy-3-butoxypropyl)-2-methylimidazole, 1-(2-hydroxy-3-butoxypropyl)-2-ethyl-4-methylimidazole, 1-(2-hydroxy-3-phenoxypropyl)-2-phenylimidazoline, 1-(2-hydroxy-3-butoxypropyl)-2-methylimidazoline, 2-(dimethylaminomethyl)phenol, 2,4,6-tris(dimethylaminomethyl)phenol, N-β-hydroxyethylmorpholine, 2-dimethylaminoethanethiol, 2-mercaptopyridine, 2-benzoimidazole, 2-mercaptobenzoimidazole, 2-mercaptobenzothiazole, 4-mercaptopyridine, N,N-dimethylaminobenzoic acid, N,N-dimethylglycine, nicotinic acid, isonicotinic acid, picolinic acid, N,N-dimethylglycine hydrazide, N,N-dimethylpropionic acid hydrazide, nicotinic acid hydrazide, and isonicotinic acid hydrazide; and the like can be mentioned.

When producing the amine adduct-based latent curing accelerator by carrying out an addition reaction between the aforementioned epoxy compound and amine compound, it is also possible to further add an active hydrogen compound having two or more active hydrogens in the molecule. As such active hydrogen compounds, for example, polyhydric phenols such as bisphenol A, bisphenol F, bisphenol S, hydroquinone, catechol, resorcinol, pyrogallol, and phenol novolac resins; polyhydric alcohols such as trimethylolpropane; polycarboxylic acids such as adipic acid and phthalic acid; 1,2-dimercaptoethane, 2-mercaptoethanol, 1-mercapto-3-phenoxy-2-propanol, mercaptoacetic acid, anthranilic acid, lactic acid, and the like can be mentioned.

As the isocyanate compound used as a production raw material for the amine adduct-based latent curing accelerator, for example, monofunctional isocyanate compounds such as butyl isocyanate, isopropyl isocyanate, phenyl isocyanate, and benzyl isocyanate; polyfunctional isocyanate compounds such as hexamethylene diisocyanate, tolylene diisocyanate (e.g., 2,4-tolylene diisocyanate, 2,6-tolylene diisocyanate), 1,5-naphthalene diisocyanate, diphenylmethane-4,4'-diisocyanate, isophorone diisocyanate, xylylene diisocyanate, paraphenylene diisocyanate, 1,3,6-hexamethylene triisocyanate, and bicycloheptane triisocyanate; furthermore, terminal isocyanate group-containing compounds obtained by the reaction of these polyfunctional isocyanate compounds and active hydrogen compounds; and the like can be mentioned. As such terminal isocyanate group-containing compounds, for example, an addition compound having a terminal isocyanate group obtained by the reaction of tolylene diisocyanate and trimethylolpropane, an addition compound having a terminal isocyanate group obtained by the reaction of tolylene diisocyanate and pentaerythritol, and the like can be mentioned.

The curing accelerator can be easily obtained, for example, by appropriately mixing the above-mentioned production raw materials, reacting them at a temperature from room temperature (25°C) to 200°C, then cooling and solidifying, followed by pulverization, or alternatively, by reacting the above-mentioned production raw materials in a solvent such as methyl ethyl ketone, dioxane, and tetrahydrofuran, removing the solvent, and then pulverizing the solid content.

As the curing accelerator, commercially available products may be used. Examples of amine-epoxy adduct-based latent curing accelerators include, for example, "AJICURE (registered trademark) PN-23" (Ajinomoto Fine-Techno Co., Inc.), "AJICURE (registered trademark) PN-H" (Ajinomoto Fine-Techno Co., Inc.), "Hardener X-3661S" (ACR Co., Ltd.), "Hardener X-3670S" (ACR Co., Ltd.), "Novacure (registered trademark) HX-3742" (Asahi Kasei Corporation), "Novacure (registered trademark) HX-3721" (Asahi Kasei Corporation), and the like, and examples of amine-isocyanate-based latent curing accelerators include, for example, "FUJICURE FXE-1000" (Fuji Kasei Kogyo Co., Ltd.), "FUJICURE FXR-1030" (Fuji Kasei Kogyo Co., Ltd.), and the like.

In the epoxy resin composition of the present invention, the content of the curing accelerator relative to 100 parts by mass of the epoxy resin is preferably 0.1 parts by mass or more, more preferably 0.5 parts by mass or more, still more preferably 1 part by mass or more, even more preferably 1.5 parts by mass or more, and particularly preferably 2 parts by mass or more. Further, the content of the curing accelerator relative to 100 parts by mass of the epoxy resin is preferably 40 parts by mass or less, more preferably 35 parts by mass or less, still more preferably 30 parts by mass or less, even more preferably 25 parts by mass or less, and particularly preferably 20 parts by mass or less. The preferred content of the curing accelerator may be within a range selected from any value among the said upper limit values, lower limit values, and values in the Examples.

As the solvent, water or organic solvents that can usually be used in the relevant field can be mentioned. As the organic solvent, toluene, tetrahydrofuran, methyl ethyl ketone, methyl isobutyl ketone, dioxane, and the like can be mentioned. The solvent may be present, but it is preferable that it is finally removed from the epoxy resin composition.

The storage stabilizer is one used for imparting excellent storage stability to the epoxy resin composition. As the storage stabilizer, it is preferable to further contain one or more selected from, for example, borate compounds, titanate compounds, aluminate compounds, zirconate compounds, isocyanate compounds, carboxylic acids, acid anhydrides, and mercapto organic acids.

Here, as the said borate compound, for example, trimethyl borate, triethyl borate, tripropyl borate, triisopropyl borate, tributyl borate, tripentyl borate, triallyl borate, trihexyl borate, tricyclohexyl borate, trioctyl borate, trinonyl borate, tridecyl borate, tridodecyl borate, trihexadecyl borate, trioctadecyl borate, tris(2-ethylhexyloxy)borane, bis(1,4,7,10-tetraoxaundecyl)(1,4,7,10,13-pentaoxatetradecyl)(1,4,7-trioxaundecyl)bora ne, tribenzyl borate, triphenyl borate, tri-o-tolyl borate, tri-m-tolyl borate, and triethanolamine borate, and the like can be mentioned.

As the said titanate compound, for example, tetraethyl titanate, tetrapropyl titanate, tetraisopropyl titanate, tetrabutyl titanate, and tetraoctyl titanate, and the like can be mentioned.

As the said aluminate compound, for example, triethyl aluminate, tripropyl aluminate, triisopropyl aluminate, tributyl aluminate, and trioctyl aluminate, and the like can be mentioned.

As the said zirconate compound, for example, tetraethyl zirconate, tetrapropyl zirconate, tetraisopropyl zirconate, and tetrabutyl zirconate, and the like can be mentioned.

As the said isocyanate compound, for example, butyl isocyanate, isopropyl isocyanate, 2-chloroethyl isocyanate, phenyl isocyanate, p-chlorophenyl isocyanate, benzyl isocyanate, hexamethylene diisocyanate, 2-ethylphenyl isocyanate, 2,6-dimethylphenyl isocyanate, tolylene diisocyanate (e.g., 2,4-tolylene diisocyanate, 2,6-tolylene diisocyanate), 1,5-naphthalene diisocyanate, diphenylmethane-4,4'-diisocyanate, tolidine diisocyanate, isophorone diisocyanate, xylylene diisocyanate, paraphenylene diisocyanate, and bicycloheptane triisocyanate, and the like can be mentioned.

As the said carboxylic acid, for example, saturated aliphatic monobasic acids such as formic acid, acetic acid, propionic acid, butyric acid, caproic acid, and caprylic acid; unsaturated aliphatic monobasic acids such as acrylic acid, methacrylic acid, and crotonic acid; halogenated fatty acids such as monochloroacetic acid and dichloroacetic acid; monobasic hydroxy acids such as glycolic acid, lactic acid, and tartaric acid; aliphatic aldehyde acids and keto acids such as glyoxylic acid; aliphatic polybasic acids such as oxalic acid, malonic acid, succinic acid, and maleic acid; aromatic monobasic acids such as benzoic acid, halogenated benzoic acid, toluic acid, phenylacetic acid, cinnamic acid, and mandelic acid; aromatic polybasic acids such as phthalic acid and trimesic acid; and the like can be mentioned.

As the said acid anhydride, for example, aliphatic polybasic acid anhydrides such as succinic anhydride, dodecenylsuccinic anhydride, maleic anhydride, adduct of methylcyclopentadiene and maleic anhydride, hexahydrophthalic anhydride, and methyltetrahydrophthalic anhydride; aromatic polybasic acid anhydrides such as phthalic anhydride, trimellitic anhydride, and pyromellitic anhydride; can be mentioned.

As the said mercapto organic acid, for example, mercapto aliphatic monocarboxylic acids such as mercaptoacetic acid, mercaptopropionic acid (e.g., 3-mercaptopropionic acid), mercaptobutyric acid (e.g., 3-mercaptobutyric acid, 4-mercaptobutyric acid); esters containing a thiol group and a carboxy group obtained by esterification reaction between a hydroxy acid and a mercapto organic acid; mercapto aliphatic dicarboxylic acids such as mercaptosuccinic acid and dimercaptosuccinic acid (e.g., 2,3-dimercaptosuccinic acid); mercapto aromatic monocarboxylic acids such as mercaptobenzoic acid (e.g., 4-mercaptobenzoic acid); and the like can be mentioned.

As the storage stabilizer, from the viewpoint of high versatility and safety, and improving storage stability, borate compounds are preferable, triethyl borate, tripropyl borate, triisopropyl borate, and tributyl borate are more preferable, and triethyl borate is still more preferable.

The content of the storage stabilizer in the epoxy resin composition of the present invention is not particularly limited as long as the storage stability of the said composition is enhanced, but relative to 100 parts by mass of the epoxy resin, the storage stabilizer is preferably 0.001 to 50 parts by mass, more preferably 0.05 to 30 parts by mass, and still more preferably 0.1 to 10 parts by mass.

The other curing agent may be any compound other than the thiol compound of the present invention described above, provided that it can be used as a curing agent for epoxy resins. As the other curing agent, for example, a complete ester of a polyol and a mercapto organic acid can be mentioned. Here, complete ester means an ester of a polyol and a carboxylic acid, in which all hydroxy groups of the polyol form ester bonds. As the said polyol, for example, ethylene glycol, trimethylolpropane, pentaerythritol, dipentaerythritol, and the like can be mentioned. As the said mercapto organic acid, for example, mercapto aliphatic monocarboxylic acids such as mercaptoacetic acid, mercaptopropionic acid (e.g., 3-mercaptopropionic acid), and mercaptobutyric acid (e.g., 3-mercaptobutyric acid, 4-mercaptobutyric acid); esters containing a thiol group and a carboxy group obtained by esterification reaction between a hydroxy acid and a mercapto organic acid; mercapto aliphatic dicarboxylic acids such as mercaptosuccinic acid and dimercaptosuccinic acid (e.g., 2,3-dimercaptosuccinic acid); mercapto aromatic monocarboxylic acids such as mercaptobenzoic acid (e.g., 4-mercaptobenzoic acid); and the like can be mentioned. The carbon number of the said mercapto aliphatic monocarboxylic acid is preferably 2 to 8, more preferably 2 to 6, still more preferably 2 to 4, and particularly preferably 3. Among the said mercapto organic acids, mercapto aliphatic monocarboxylic acids having 2 to 8 carbon atoms are preferable, mercaptoacetic acid, 3-mercaptopropionic acid, 3-mercaptobutyric acid, and 4-mercaptobutyric acid are more preferable, and 3-mercaptopropionic acid is still more preferable.

Specific examples of complete esters of polyols and mercapto organic acids include ethylene glycol bis(mercaptoacetate), ethylene glycol bis(3-mercaptopropionate), ethylene glycol bis(3-mercaptobutyrate), ethylene glycol bis(4-mercaptobutyrate), trimethylolpropane tris(mercaptoacetate), trimethylolpropane tris(3-mercaptopropionate), trimethylolpropane tris(3-mercaptobutyrate), trimethylolpropane tris(4-mercaptobutyrate), pentaerythritol tetrakis(mercaptoacetate), pentaerythritol tetrakis(3-mercaptopropionate), pentaerythritol tetrakis(3-mercaptobutyrate), pentaerythritol tetrakis(4-mercaptobutyrate), dipentaerythritol hexakis(mercaptoacetate), dipentaerythritol hexakis(3-mercaptopropionate), dipentaerythritol hexakis(3-mercaptobutyrate), and dipentaerythritol hexakis(4-mercaptobutyrate), and the like. Further, from the viewpoint of storage stability, the said complete ester preferably has a basic impurity content as low as possible, and one that does not require the use of basic substances in production is more preferable.

Further, as other curing agents, alkyl polythiol compounds such as 1,4-butanedithiol, 1,6-hexanedithiol, and 1,10-decanedithiol; terminal thiol group-containing polyethers; terminal thiol group-containing polythioethers; polythiol compounds obtained by reaction between an epoxy compound and hydrogen sulfide; polythiol compounds having terminal thiol groups obtained by reaction between a polythiol compound and an epoxy compound; polythiol compounds produced using a basic substance as a reaction catalyst in their production process, such as the foregoing, can also be used. It is preferable to use the polythiol compound produced using a basic substance after performing alkali removal treatment to reduce the alkali metal ion concentration to 50 ppm by weight or less. Examples of the alkali removal treatment for polythiol compounds produced using a basic substance include a method of desalting by dissolving the polythiol compound in an organic solvent such as acetone and methanol, neutralizing by adding an acid such as dilute hydrochloric acid and dilute sulfuric acid, followed by extraction, washing, etc.; a method of adsorbing using an ion-exchange resin; a method of purifying by distillation; and the like.

Further, as other curing agents, for example, tris[(3-mercaptopropionyloxy)-ethyl]-isocyanurate, 1,3,5-tris(3-mercaptobutyloxyethyl)-1,3,5-triazine-2,4,6(1H,3H,5H)-trione, etc., can be used. Preferably, it is one or more of complete esters of ethylene glycol, trimethylolpropane, pentaerythritol or dipentaerythritol and a mercapto aliphatic monocarboxylic acid having 2 to 8 carbon atoms; more preferably, it is at least one selected from ethylene glycol bis(mercaptoacetate), ethylene glycol bis(3-mercaptopropionate), ethylene glycol bis(3-mercaptobutyrate), ethylene glycol bis(4-mercaptobutyrate), trimethylolpropane tris(mercaptoacetate), trimethylolpropane tris(3-mercaptopropionate), trimethylolpropane tris(3-mercaptobutyrate), trimethylolpropane tris(4-mercaptobutyrate), pentaerythritol tetrakis(mercaptoacetate), pentaerythritol tetrakis(3-mercaptopropionate), pentaerythritol tetrakis(3-mercaptobutyrate), pentaerythritol tetrakis(4-mercaptobutyrate), dipentaerythritol hexakis(mercaptoacetate), dipentaerythritol hexakis(3-mercaptopropionate), dipentaerythritol hexakis(3-mercaptobutyrate), and dipentaerythritol hexakis(4-mercaptobutyrate); still more preferably, it is at least one selected from trimethylolpropane tris(mercaptoacetate), trimethylolpropane tris(3-mercaptopropionate), trimethylolpropane tris(3-mercaptobutyrate), and trimethylolpropane tris(4-mercaptobutyrate); particularly preferably it is trimethylolpropane tris(3-mercaptopropionate).

When using another curing agent in the epoxy resin composition, the amount is preferably in a range that does not affect the curing of the thiol compound of the present invention, but relative to 100 parts by mass of the epoxy resin, the other curing agent is preferably 0.001 to 50 parts by mass, more preferably 0.05 to 30 parts by mass, and still more preferably 0.1 to 10 parts by mass.

The ratio of the thiol compound of the present invention to the other curing agent, [mass of the thiol compound of the present invention]/[total mass of the thiol compound of the present invention and the other curing agent], is preferably, for example, 0.001 to 0.8. The lower limit of this ratio is more preferably 0.01, still more preferably 0.03, and particularly preferably 0.1. The upper limit of this ratio is not particularly limited, but is more preferably 0.6, still more preferably 0.4, and particularly preferably 0.2.

The ratio of the total equivalent of thiol groups contained in the thiol compound of the present invention and/or the other curing agent to the equivalent of epoxy groups contained in the epoxy resin, i.e., [total number of thiol groups contained in the thiol compound of the present invention and/or the other curing agent]/[number of epoxy groups contained in the epoxy resin], is preferably 0.2 to 2.0, more preferably 0.6 to 1.2.

As the thixotropy-imparting agent, for example, precipitated silica, calcined silica, and fumed silica (fumed silica), etc., can be mentioned, and among these, fumed silica (fumed silica) is preferable. The specific surface area by the BET method (hereinafter referred to as BET specific surface area) of the thixotropy-imparting agent is preferably 50 m²/g or more, more preferably 50 to 600 m²/g, still more preferably 100 to 400 m²/g. When using a thixotropy-imparting agent, the content of the thixotropy-imparting agent in the epoxy resin composition, relative to 100 parts by mass of the epoxy resin, is preferably 5 to 200 parts by mass, more preferably 10 to 100 parts by mass for the thixotropy-imparting agent.

The epoxy resin composition may be a one-component type epoxy resin composition or a two-component type epoxy resin composition. In the case of a two-component type epoxy resin composition, for example, it may be a kit having agent A and agent B, where the epoxy resin is agent A, and the thiol compound and other additives are agent B. Further, the epoxy resin composition may contain one type or multiple types of each component such as the above-mentioned thiol compound.

### [Epoxy Resin Cured Product]

One aspect of the present invention can be an epoxy resin cured product formed by curing the above-mentioned epoxy resin in the presence of the above-mentioned thiol compound. As the curing method, a thermosetting method usually used for epoxy resins is used. For example, an epoxy resin, a thiol compound, and other optional additives may be mixed to prepare an epoxy resin composition, and the said epoxy resin composition may be maintained, for example, at a temperature of 50 to 250°C, preferably 80 to 200°C, more preferably 120°C±10 to 20°C, for example, for 10 minutes to 3 hours, preferably 30 minutes to 2 hours, more preferably for 1 hour±10 to 20 minutes, to obtain a cured product. At the time of preparing the epoxy resin composition, it is preferable to deaerate each component using a deaerator or the like.

The epoxy resin cured product of the present invention can be used for applications such as, for example, adhesives, casting agents, sealing agents, encapsulants, resins for fiber reinforcement, coating agents, and paints.

### Examples

Hereinafter, the present invention will be explained more specifically based on Examples and Comparative Examples, but the present invention is not limited to the following Examples.

### [Method for Measuring High-Performance Liquid Chromatography and Mass Spectrum (LC/MS Measurement)]

### (Measurement Conditions for High-Performance Liquid Chromatography and Mass Spectrum)

A sample was diluted to 1 mg/mL with tetrahydrofuran (THF), and measurement of high-performance liquid chromatography and mass spectrum (LC/MS) was performed under the following conditions.
High-Performance Liquid Chromatography (HPLC): ACQUITY UPLC (manufactured by Waters Corporation)
Mass Spectrum (MS): SQ Detector 2 (manufactured by Waters Corporation)
Column: ACQUITY UPLC BEH C8 1.7um, 2.1mm x 50mm (manufactured by Waters Corporation)
Mobile phase A: 2 mmol ammonium acetate aqueous solution
Mobile phase B: 2-Propanol/acetonitrile (50:50)
Mobile phase mixing time and mixing ratio (A%): 0 to 0.5 min (95%) -> 1 min to 8.5 min (75%) -> 9 to 11 min (5%) -> 11.1 min (95%) -> 13 min (95%)
Flow rate: 0.30 mL/min
Analysis time: 13 minutes
Column temperature: 40°C, Ion mode: ESI (Electrospray Ionization Method) Positive
Ion polarity: Positive detection mode
Desolvation gas flow rate: 700 L/hr, 250°C
Cone gas: 70 L/hr
Ion source heater: 150°C

### [Method for Measuring NMR]

NMR data for thiol compounds (1) and (2) prepared in Examples 1 and 2 was collected using JEOL LA-500 (manufactured by JEOL Ltd.).

### [Example 1]

### Synthesis of Thiol Compound (1)

### (1) Synthesis of Thioester Body A

Into a 200 mL four-necked flask were charged tetraallyloxyethane (below, 20 g, 0.078 mol, 1.0 eq), toluene (100 mL), azobisisobutyronitrile (AIBN, 1.29 g, 0.008 mol, 0.1 eq), and under a nitrogen atmosphere, thioacetic acid (26.34 g, 0.346 mol, 4.4 eq) was added dropwise, and reacted at 80°C for 3 hours. Next, toluene was removed by concentration under reduced pressure to obtain 45.0 g of yellow liquid thioester body A (below).

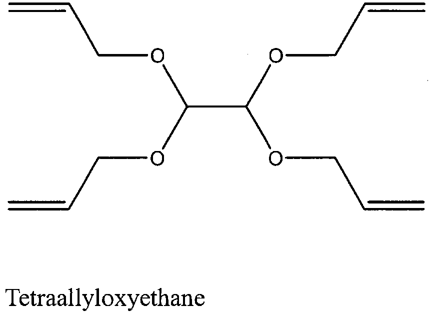

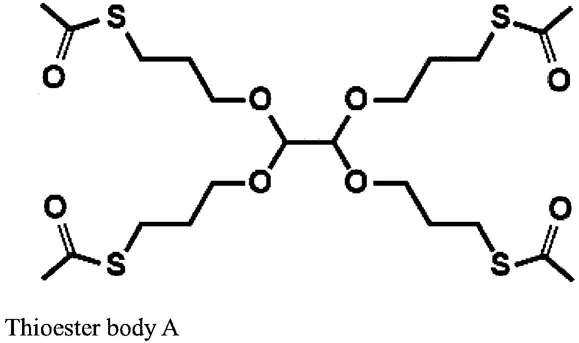

### (2) Synthesis of Thiol Compound

Next, into a 500 mL four-necked flask were charged thioester body A obtained in the above (1) (40 g), methanol (200 g), and 20% NaOH (20.2 g), and reacted under a nitrogen atmosphere at 70°C for 1.5 hours. Next, after adding 2.5N HCl (30 mL) for neutralization, extraction was performed 3 times with toluene (50 mL). After washing the extracted organic layer with saturated brine, dehydration was performed using magnesium sulfate, and insoluble matter was filtered off by filtration. Toluene was removed from the organic layer of the filtrate by concentration under reduced pressure, obtaining 27.2 g of yellow liquid thiol compound (LC purity 58.3%, yield 97.3%).

As a result of performing measurement on the obtained thiol compound according to the LC/MS measurement method described above, spectral peaks corresponding to a proton adduct m/z=391 and corresponding to an ammonium adduct m/z=408 were detected. From the said analysis data and the results of the NMR spectrum shown in Fig. 1, it was confirmed that the obtained thiol compound was thiol compound (1) pertaining to the structure below.

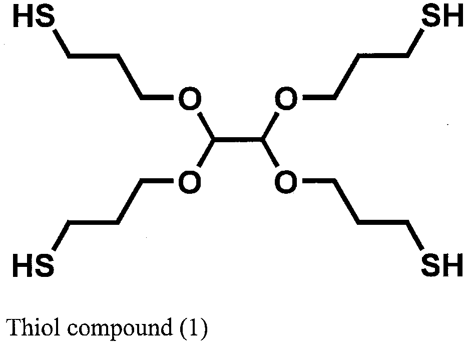

### [Example 2]

### Synthesis of Thiol Compound (2)

### (1) Synthesis of Thioester Body B

Thioester body B below was obtained in the same manner as in Example 1 except that 20 g (0.078 mol) of tetraallyloxyethane of Example 1 was changed to 24.2 g (0.078 mol) of tetra(2-methylallyl)oxyethane (below).

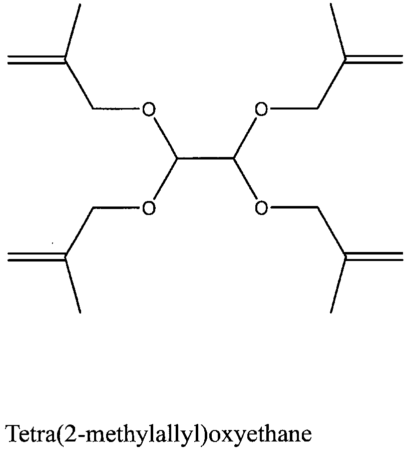

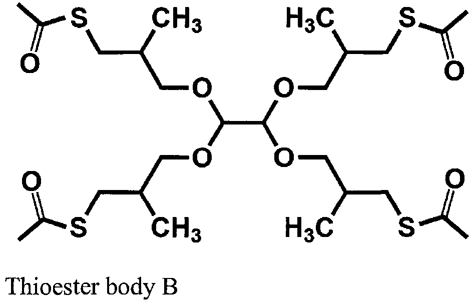

### (2) Synthesis of Thiol Compound

Thioester body B obtained in the above (1) was subjected to reaction etc. in the same manner as in Example 1(2), obtaining 28 g of yellow liquid thiol compound.

As a result of performing measurement on the obtained thiol compound according to the LC/MS measurement method described, spectral peaks corresponding to a proton adduct m/z=447 and corresponding to an ammonium adduct m/z=464 were detected. From the said analysis data, it was confirmed that the obtained thiol compound was thiol compound (2) pertaining to the structure below.

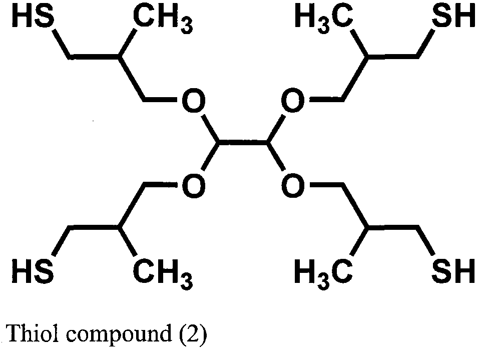

### [Evaluation Test]

The curing state when an epoxy resin was cured using the thiol compounds obtained in Examples 1 and 2 above was evaluated. Specifically, the amounts (component amounts in Table 1 are parts by mass) shown in Table 1 below of epoxy resin, thiol compound, and curing accelerator (AJICURE (registered trademark) PN-H, manufactured by Ajinomoto Fine-Techno Co., Inc.) were weighed and mixed. Thereafter, the mixture was deaerated under vacuum using an automatic planetary stirring deaerator (manufactured by Kyoritsu Seiki Co., Ltd.: HM-200W) at 900 rpm for 2 minutes, to obtain the target resin composition. The obtained resin composition was cured in a 120°C thermal circulation oven for 1 hour, to obtain a cured product. The surface of the obtained said cured product was touched with a finger, and it was checked whether there was any adhering matter on the finger.

As the epoxy resin, jER (registered trademark) 828 (manufactured by Mitsubishi Chemical Corporation) was used. As the thiol curing agent of Comparative Example 1, PE-1 (pentaerythritol tetrakis(3-mercaptobutyrate), manufactured by Resonac Corporation) was used.

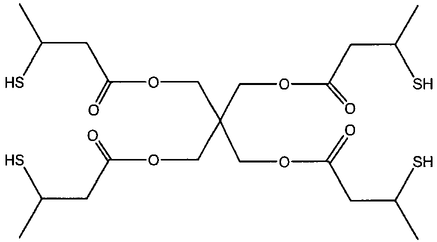

### PE-1 (pentaerythritol tetrakis(3-mercaptobutyrate))

**Table 1**

| Component (parts by mass) | | Example 1 | Example 2 | Comparative Example 1 |
|---|---|---|---|---|
| Epoxy resin | jER828 | 100 | 100 | 100 |
| Curing agent | Thiol compound (1) | 57 | | |
| | Thiol compound (2) | | 57 | |
| | PE-1 | | | 57 |
| Curing accelerator | PN-H | 5 | 5 | 5 |
| Tack-free property evaluation result after curing at 120°C for 1 hour | | Tack-free, cured, flexible | Tack-free, cured, flexible | Tack-free, cured, elastic body |

As shown in Table 1, in Examples 1 and 2, flexible cured products without tackiness were obtained. On the other hand, in Comparative Example 1, a cured product without tackiness was obtained, but a hard elastic body cured product was obtained.

## Claims

1. A compound represented by the general formula (I): 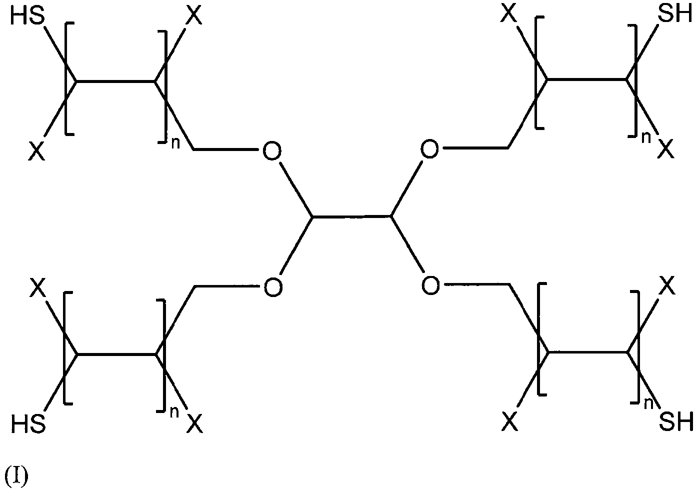 (in the formula (I), X is, each independently, a hydrogen atom, a C1-C6 alkyl group, a C1-C6 haloalkyl group, or a C1-C6 alkoxy group, and n is, each independently, an integer of 1 to 5).

2. The compound according to claim 1, wherein X is, each independently, a hydrogen atom, a methyl group, or an ethyl group, and n is an integer of 1 to 3.

3. The compound according to claim 1, wherein X is, each independently, a hydrogen atom or a methyl group, and n is 1.

4. A compound shown below. 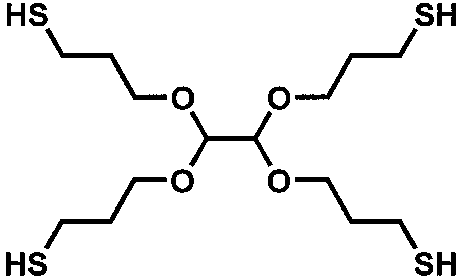 or

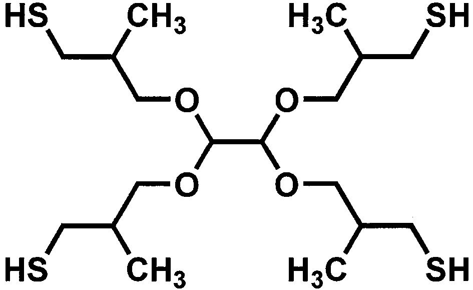

5. An epoxy resin composition, comprising an epoxy resin and the compound according to any one of claims 1 to 4.

6. A curing agent for epoxy resins, comprising the compound according to any one of claims 1 to 4.

7. An epoxy resin cured product, formed by thermosetting an epoxy resin and the compound according to any one of claims 1 to 4.
